# EUROPEAN PATENT APPLICATION

(11) **EP 4 487 876 A1**
(43) Date of publication of application: **08.01.2025**
(21) Application number: 23184323.6
(22) Date of filing: 07.07.2023
(51) Int. Cl.: A61L 15/26, A61L 15/42

(54) **IMPROVED WOUND DRESSING AND METHOD TO PREPARE IT**

(71) Applicant: Leibniz-Institut für Polymerforschung Dresden e.V., 01069 Dresden (DE)
(72) Inventor: FREUDENBERG, Uwe, 01217 Dresden (DE); SCHIRMER, Lucas, 01217 Dresden (DE); ATALLAH, Passant, 01099 Dresden (DE); KÜHN, Sebastian, 01099 Dresden (DE); WERNER, Carsten, 01324 Dresden (DE)
(74) Representative: Schrell, Andreas

(57) **Abstract**

The present invention relates to a wound dressing comprising a polymeric support having a coating of a polymer network comprising a poly-(4-styrenesulfonic acid-co-maleic acid) (PSS-MA) component covalently bonded to at least one of an uncharged polymer component and a non-polymeric crosslinker component thereby forming the polymer network, wherein the PSS-MA component comprises sulfonate groups, and a method to prepare it.

## Description

The present invention relates to a wound dressing comprising a polymeric support having a coating of a polymer network comprising a poly-(4-styrenesulfonic acid-co-maleic acid) component covalently bonded to at least one of an uncharged polymer component and a non-polymeric crosslinking component thereby forming the polymer network, wherein the PSS-MA component comprises sulfonate groups, and a method to prepare it.

In Germany, more than 2.7 million patients suffer from chronic, non-healing wounds. The treatment of these wounds can take months to years and requires frequent visits to the doctor and nursing care. With an aging population and increasing incidence of diabetes and obesity, many more patients will suffer from hard-to-heal wounds in the future. The main cause of delayed wound healing is a prolonged and intensified inflammatory phase, which prevents the onset of healing. Accordingly, a multitude of various therapeutic applications have been developed so as to effectively cure wounds.

EP 1 718 257 B1 discloses a multi layered wound dressing for highly exudating wounds, which includes a transmission layer having high moisture vapor transmission rate, absorbent core, and a wound contacting layer.

EP 1 988 909 B1 discloses a hydrogel composition or aqueous solution useful in the treatment of inflammation of the skin or mucosa and as a dermatological product, which comprises poly(ethylene glycol) having specific molecular weight.

EP 2 736 486 A1 discloses a composition useful for treating a wound, which comprises a pharmaceutically acceptable carrier, an effective amount of an active ingredient of inorganic solids, and an antimicrobial biguanide compound.

EP 2 742 997 B1 discloses a carrier used for for instance inflammatory cytokine adsorption, which comprises high molecular com-pound(s) having two or more repeating units containing aromatic ring which is covalently bonded through specific structure.

WO 2021/198464 A1 discloses a hydrogel-based wound dressing formulation useful for treating wound, which comprises a copolymer of monomers with at least one covalently linked multifunctional group, and a source of nitrite and/or nitrate.

WO 2021/212154 A1 discloses a wound dressing useful for treating wound, which comprises a substrate having first surface and second surface comprising a sulfonated polymeric layer containing sulfonated polymer.

The known technologies often use specific substances which are classified as pharmaceuticals and thus frequently cause from side-effects and need to be admitted in costly and time-consuming proceedings by the competent drug authorities prior to use. Other technologies do not exert the desired wound healing effects in a satisfactory manner or are complicated and expensive in production, structure and/or use.

Thus, there is an urgent need to provide means and methods to improve wound healing, in particular to allow a rapid wound healing, in particular with a reduced inflammatory phase, in particular in a cost-effective and simple manner, in particular wherein the means and methods do not rely on the use of pharmaceutically active agents.

The technical problem underlying the present invention therefore is to overcome the aforementioned disadvantages.

Thus, the technical problem underlying the present invention is in particular to provide means and methods to improve the wound healing process in subjects in need thereof, in particular humans and animals, in particular means and methods which allow a faster wound healing and reduce the duration and/or intensity of the inflammatory phase, in particular wherein the means and methods do not rely on the use of pharmaceutically active agents.

The technical problem is solved by the teaching of the present invention, in particular the teaching as specified in the independent and dependent claims as well as in the present description.

Thus, the present invention in particular provides a wound dressing comprising a polymeric support having a coating of a polymer network, the polymer network comprising at least one poly-(4-styrenesulfonic acid-co-maleic acid) (in the following also termed PSS-MA) component covalently bonded to at least one of at least one uncharged polymer component and at least one non-polymeric crosslinking component thereby forming the polymer network, wherein the PSS-MA component comprises sulfonate groups.

The PSS-MA component in the polymer network comprises sulfonate groups, that means free, unbound and ionizable, functional groups which are preferably negatively charged, in particular under physiological conditions, that means under the condition of being applied to a wound, and which are, thus, not covalently bond to other residues or entities of the polymeric support or the polymer network.

Without being bound by theory, the sulfonate groups in the polymer network are preferably essentially in deprotonated form. These deprotonated sulfonate groups can undergo charge compensation or form charge interactions by counterions and/or proteins. The sulfonate groups may in some embodiments be in protonated form.

These sulfonate groups are accordingly and preferably in the polymer network present in form of anionic groups and are thus able to interact based on the electrical charge, with cationic groups, and therefore are capable to bind and release positively charged molecules, in particular chemokines. According to the invention, these sulfonate groups are not covalently bonded to the uncharged polymer component, in particular not to the preferred poly(ethylene glycol) (PEG) component. According to the invention, these sulfonate groups are not covalently bonded to the non-polymeric crosslinking component. The PSS-MA used to prepare the polymer network comprises also carboxylate or carboxyl groups, which, however, are primarily used to provide covalent bonds to other components of the polymer network, in particular to the uncharged polymer component and/or the non-polymeric crosslinking component. Thus, at least most of the carboxylate or carboxyl groups of the PSS-MA component are no free groups but participate in covalently binding components of the polymer network to each other.

Thus, the present invention provides a wound dressing comprising, in particular consisting of, a polymeric support and a coating thereof, which coating is comprising, in particular consisting of, a polymer network as specified herein. The polymer network preferably coats (hereinafter also referred to as "surrounds") the polymeric support completely or partially, that means the polymer network constitutes a complete or partial, preferably complete, coating of the surface of the polymeric support, in particular of its fibers and/or mesh or foam structures.

The polymeric support (hereinafter also termed to be a "carrier") may be a polymeric foam, a textile or fabric, in particular be a mesh, a nonwoven fabric, a woven fabric, a knitted fabric, a perforated film, a fiber or a fiber mesh.

The polymer-coated wound dressing of the present invention protects the wound from physical influences of an optionally applied secondary wound dressing and minimizes irritation of the wound during dressing change. As a result, it leads to relief of patient pain and discomfort, and minimizes trauma to the wound and surrounding skin during dressing changes. The main effect of the polymeric support is its function as a wound spacer being achieved by creating a mechanical barrier between the wound and an additionally and optionally applied secondary wound dressing. The polymeric support protects the wound from sticking to the secondary wound dressing, thereby enabling atraumatic wound dressing changes and, at the same time, its meshed or foam structure also ensures the removal of excess wound exudate and its absorption by the secondary wound dressing or the polymeric foam-support itself.

The wound dressing very advantageously comprises a unique polymer coating, that means the polymer network, on the polymeric support, which promotes the causal resolution of the chronic inflammatory process, thus supporting wound closure and enabling a rapid wound healing. This shortens the duration of inflammation, pain and discomfort and prevents further damage to the surrounding skin and other tissues as well as a potential consequential damage such as an amputation.

The polymer coating of the polymeric support, which comprises, in particular consists of, at least one PSS-MA component and covalently cross-linked thereto at least one of at least one uncharged polymer component, in particular a star-PEG, and at least one non-polymeric crosslinking component ensures hydrophilization of the polymeric support through water retention in the polymer network and prevents the wound from sticking to the support. In addition, the at least one PSS-MA component, covalently bound in the polymer coating that means the polymer network, promotes wound healing by physically binding pro-inflammatory substances, in particular chemokines, and withdrawing them from the wound area. The chronic inflammatory process can thus be stopped and the transition to secondary wound healing thus enabled. Thus, a physical adsorption process takes place that brings about this effect and is based on charge interactions between the negatively charged sulfonate of the PSS-MA component and positively charged amino acid residues of the pro-inflammatory chemokines.

The main effect of the present wound dressing as a polymer-coated wound spacer grid is therefore a) in the mechanical barrier function to an optional secondary wound dressing preventing the wound from sticking to the secondary wound dressing, b) in enabling fluid management of the wound, that means excess wound secretion can be drained off and absorbed by a secondary wound dressing, and c) the physical process of binding excess, pro-inflammatory substances in particular chemokines, and removing them from the wound area, brought about by the PSS-MA component covalently bound in the polymer coating thereby providing a covalently crosslinked, hydratable polymer network.

According to one embodiment of the invention, the at least one uncharged polymer component is covalently bonded to the at least one PSS-MA component, in particular directly or by means of a crosslinking component. The uncharged polymer component and the PSS-MA component covalently bonded thereto thus together, optionally with the additional presence of the non-polymeric crosslinking component, form the polymer network.

According to the invention, in a further embodiment, the at least one non-polymeric crosslinking component is covalently bonded to the at least one PSS-MA component, in particular directly. The at least one non-polymeric crosslinking component and the PSS-MA component covalently bonded thereto, thus, together form the polymer network.

The invention therefore provides in particular that the at least one PSS-MA component is covalently bonded directly or via at least one crosslinking component to the at least one non-charged polymer component, forming the polymer network, or, in a further embodiment, that the at least one PSS-MA component is bonded to the at least one crosslinking component, forming the polymer network.

In a preferred embodiment, PSS-MA may have a MW of 10 000 to 40 000 g/mol, in particular 20 000 to 30 000 g /mol, preferably 20 000 to 21 000 g/mol.

In a preferred embodiment, the PSS-MA is a copolymer with a molar ratio from 5:1 to 1:1, in particular 3:1 to 1:1 of 4-styrenesulfonic acid : maleic acid.

The PSS-MA used for the preparation of the polymer network comprises, in addition to sulfonate groups, carboxylate or carboxyl groups, with which the PSS-MA is covalently bonded to the uncharged polymer and/or the non-polymeric crosslinking component used to form the polymer network, such that the network is formed having at least one PSS-MA component crosslinked via at least one uncharged polymer component or at least one non-polymeric crosslinking component or crosslinked via both to form a network.

In a particularly preferred embodiment, the at least one uncharged polymer component is selected from the group consisting of poly(ethylene glycol) (PEG) component, poly(2-oxazoline) (POX) component, polyvinylpyrrolidone (PVP) component, poly(vinyl alcohol) (PVA) component, polyacrylamide (PAM) component, and combinations thereof.

The uncharged polymer may be linear or branched, in particular multi-arm, that is, this comprises several branched chains in a preferred embodiment of the present invention. In particular, the uncharged polymer is star-shaped, that is, multi-arm with a centre from which a plurality of chains, in particular four or eight chains, in particular four chains of equal length, branch off from corresponding repeating units.

In a preferred embodiment, the at least one uncharged polymer has an average molecular weight of from 5 000 to 25 000, in particular from 10 000 to 19 000 Da.

In a preferred embodiment, the repeating unit of the at least one uncharged polymer component has an average molecular weight of from 30 to 55, in particular from 40 to 50 Da.

In a particularly preferred embodiment, the at least one uncharged polymer component is a linear uncharged polymer component.

In a particularly preferred embodiment, the at least one uncharged polymer component is a branched, in particular multi-arm, uncharged polymer component.

In a particularly preferred embodiment, the at least one multi-arm uncharged polymer component is a four-armed or eight-arm uncharged polymer component.

In a particularly preferred embodiment, the at least one multi-arm uncharged polymer component is a PEG component.

In a particularly preferred embodiment, the at least one uncharged polymer component is a poly(ethylene glycol) component, particularly a linear or multi-arm poly(ethylene glycol) component.

In a particularly preferred embodiment, the at least one multi-arm uncharged polymer component is a four-arm poly(ethylene glycol) component.

In another preferred embodiment, the at least one multi-arm uncharged polymer component is an eight-arm poly(ethylene glycol) component.

In a particularly preferred embodiment, the at least one multi-arm uncharged polymer component is a four-arm PEG. A multi-arm PEG, in particular a four-arm PEG, is hereinafter also called a starPEG.

In a preferred embodiment, starPEG may have a molecular weight (MW) from 6 000 to 20 000 g/mol, preferably 10 000 to 16 000 g/mol.

In a preferred embodiment of the present invention, the uncharged polymer used to prepare the polymer network has a functional group, in particular an end group, at the respective free end of the polymer selected from the group consisting of amino group, thiol group, maleimide group, vinylsulfone group, acrylate group, carboxyl group and combinations thereof. The uncharged polymer is thus functionalized, in particular end group functionalized. Via these reactive functional groups, in particular end groups, the at least one uncharged polymer is preferably linked to the at least one PSS-MA, thus forming the polymer network. The at least two functional groups of the uncharged polymer may be the same or different, in particular they are the same.

In a particularly preferred embodiment, the uncharged polymer used for preparing the polymer network according to the invention has at least two amino groups, in particular terminal amino groups.

In a particularly preferred embodiment, the uncharged polymer used for the production of the polymer network according to the invention has at least two carboxyl groups, in particular terminal carboxyl groups.

In a particularly preferred embodiment, the uncharged polymer, which is in particular end group functionalized with one carboxyl group each, is a multi-armed, in particular eight-armed, PEG.

In a particularly preferred embodiment, the uncharged polymer, which is in particular end-group-functionalized with one amino group in each case, is a multi-armed, in particular four-armed, PEG.

In a preferred embodiment of the present invention, the non-polymeric crosslinking component used for the preparation of the polymer network according to the invention has at least two functional groups suitable for forming a covalent bond to the PSS-MA component.

In preferred embodiments of the present invention, the non-polymeric crosslinking molecule used to prepare the polymer network has at least two functional groups, in particular end groups, selected from the group consisting of amino group, thiol group, maleimide group, vinylsulfone group, acrylate group, carboxyl group, hydroxylated aromatic group and combinations thereof. The non-polymeric crosslinking molecule is thus functionalized, particularly end group functionalized. Via these reactive functional groups, in particular end groups, the at least one non-polymeric crosslinking molecule is preferentially linked to the at least one PSS-MA, thus forming the polymer network. The at least two functional groups of the non-polymeric crosslinking molecule may be the same or different, in particular they are the same.

In a preferred embodiment, the crosslinking molecule is a non-polymeric bifunctional crosslinking molecule.

In a preferred embodiment, the crosslinking molecule has at least two amino groups. In a particularly preferred embodiment, the crosslinking molecule having at least two amino groups is selected from the group consisting of ethylenediamine, propylenediamine (1,3-diaminopropane), butane-1,4-diamine, pentane-1,5-diamine (cadaverines), hexamethylene-1,6-diamine, and combinations thereof.

In a preferred embodiment, the crosslinking molecule has at least two carboxyl groups. In a particularly preferred embodiment, the crosslinking molecule having at least two carboxyl groups is selected from the group consisting of oxalic acid, malonic acid, succinic acid, glutaric acid, adipic acid, pimelic acid, and combinations thereof.

In a particularly preferred embodiment, the crosslinking molecule is a molecule having at least two different functional groups, in particular at least two functional groups capable of crosslinking the PSS-MA component and the uncharged polymer component, in particular N-(2-aminoethyl) maleimide.

In a preferred embodiment, the at least one non-polymeric crosslinking component is selected from the group consisting of ethylenediamine, propylenediamine (1,3-diaminopropane), butane-1,4-diamine, pentane-1,5-diamine (cadaverine), hexamethylene-1,6-diamine oxalic acid, malonic acid, succinic acid, glutaric acid, adipic acid, pimelic acid, N-(2-aminoethyl) maleimide, an enzymatically cleavable peptide and combinations thereof.

In a preferred embodiment of the present invention, the uncharged polymer components and the PSS-MA components are covalently bonded by means of at least one non-polymeric crosslinking component, in particular an enzymatically cleavable peptide.

In a preferred embodiment, the starPEG may be a starPEG peptide conjugate, preferably wherein the peptides carry active thiol groups on the cysteine for crosslinking.

Preferably, for providing enzymatically cleavable polymer network, starPEG-mal can be pre-functionalized with enzymatic cleavable peptides carrying cysteine with free thiol groups at the end of the peptide sequences allowing for the crosslinking via reaction with maleimide prefunctionalized PSS-MA.

In a particularly preferred embodiment, the crosslinking molecule is in particular an enzymatically cleavable sequence, in particular a matrix metalloproteases (MMPs)-responsive element, cathepsin-responsive element, elastase-responsive element, blood coagulation enzyme-responsive element, for example thrombin-responsive GGF-pipecolic acid, FXa-responsive element, calicrein-responsive element or bacterial protease-responsive element, for example aureolysinresponsive or elastase-responsive element, or the protease IV-responsive sequence.

In a preferred embodiment, the enzymatically cleavable sequences are each flanked at the C- and N-terminal ends by a cysteine. The PSS-MA and the uncharged polymer or at least two PSS-MA are crosslinked via the respective cysteine.

In a preferred embodiment, the PSS-MA component used for the preparation of the polymer network according to the invention is covalently linked to a non-polymeric crosslinking component, in particular peptide.

In a preferred embodiment, the uncharged polymer component preferably used for the preparation of the polymer network according to the invention and the PSS-MA component are covalently linked to each other by means of a non-polymeric crosslinking component, in particular peptide.

In a preferred embodiment, the non-polymeric crosslinking component and the PSS-MA component are covalently linked to each other via at least one amide bond.

In a preferred embodiment, the uncharged polymer component and the PSS-MA component are covalently linked to each other via at least one amide bond by means of a non-polymeric crosslinking component.

In another preferred embodiment, the uncharged polymer component and the PSS-MA component used to prepare the polymer network of the invention are directly covalently bonded to one another, in particular by means of an amide bond, thiol-amine bond, disulfide bond, thioether bond, thiol-maleimide bond, preferably bioorthogonal thioether bond obtainable by thiol-maleimide, thiol-vinyl sulfone or thiol-acrylate reaction.

In a preferred embodiment, the uncharged polymer component, in particular uncharged polymer component containing amino groups, and the PSS-MA component containing carboxyl groups, used for the preparation of the polymer network according to the invention are covalently bonded directly to one another by means of an amide bond. In a preferred embodiment, the amide bond is facilitated by means of 1-ethyl-3-(3-dimethylaminopropyl) carbodiimide/N-hydroxysulfosuccinimide (EDC/sNHS) activation at the carboxyl groups of the PSS-MA component.

In a further preferred embodiment, the non-polymeric crosslinking component used for the preparation of the polymer network according to the invention, and the PSS-MA component are directly covalently bonded to each other, in particular by means of an amide bond, thiol-amine bond, disulfide bond, thioether bond, thiol-maleimide bond, preferably bioorthogonal thioether bond obtainable by thiol-maleimide, thiol-vinyl sulfone or thiol-acrylate reaction.

In a preferred embodiment, the non-polymeric crosslinking component, in particular non-polymeric crosslinking component containing amino groups, and the PSS-MA containing carboxyl groups used for the preparation of the polymer network according to the invention are covalently bonded directly to one another by means of an amide bond. In a preferred embodiment, the amide bond is facilitated by means of 1-ethyl-3-(3-dimethylaminopropyl) carbodiimide/N-hydroxysulfosuccinimide (EDC/sNHS) activation at the carboxyl groups of the PSS-MA component.

In a preferred embodiment, the polymer network comprises at least two, in particular two, different non-polymeric crosslinking components. In a particularly preferred embodiment, the polymer network has a non-polymeric crosslinking component which has at least two carboxyl groups, in particular is end-group-functionalized with one carboxyl group in each case, and a non-polymeric crosslinking component which has at least two amino groups, in particular is end-group-functionalized with one amino group in each case. Preferably, the non-polymeric crosslinking component having at least two carboxyl groups can be linked via at least one amide bond to the non-polymeric crosslinking component preferably having amino groups, and the non-polymeric crosslinking component having at least two amino groups can be linked via at least one amide bond to the PSS-MA component having carboxyl groups, wherein in a preferred embodiment the amide bond is enabled by means of 1-ethyl-3-(3-dimethylaminopropyl)carbodiimide/N-hydroxysulfosuccinimide (EDC/sNHS) activation at the carboxyl groups of the non-polymeric crosslinking molecule and the PSS-MA component.

In a preferred embodiment, the polymer network comprises at least two, in particular two, different uncharged polymer components. In a particularly preferred embodiment, the polymer network comprises an uncharged polymer component which has at least two carboxyl groups, in particular is end-group-functionalized with one carboxyl group in each case, and an uncharged polymer component which comprises at least two amino groups, in particular is end-group-functionalized with one amino group in each case. Preferably, the uncharged polymer component having at least two carboxyl groups can be connected via at least one amide bond to the uncharged polymer component preferably having amino groups, and the uncharged polymer component having at least two amino groups can be connected via at least one amide bond to the PSS-MA component having carboxyl groups, wherein in a preferred embodiment the amide bond is enabled by means of 1-ethyl-3-(3-dimethylaminopropyl)carbodiimide/N-hydroxysulfosuccinimide (EDC/sNHS) activation at the carboxyl groups of the uncharged polymer component and the PSS-MA component.

In a preferred embodiment, the polymer network comprises an uncharged polymer component. In a particularly preferred embodiment, the polymer network has an uncharged polymer component that has at least two amino groups, in particular is end-group functionalized with one amino group each. Preferably, the uncharged polymer component having at least two amino groups can be linked via at least one amide bond to the PSS-MA having carboxyl groups, wherein in a preferred embodiment the amide bond is enabled by means of 1-ethyl-3-(3-dimethylaminopropyl) carbodiimide/N-hydroxysulfosuccinimide (EDC/sNHS) activation at the carboxyl groups of the PSS-MA component.

In a particularly preferred embodiment, the polymer network, in particular in the swollen state, has sulfonate groups in a concentration of at least 0.1 mmol/l, in particular at least 1 mmol/l, in particular at least 10 mmol/l, in particular at least 100 mmol/l, in particular 0.1 to 800 mmol/l, in particular 10 to 800 mmol/l, in particular 1 to 100 mmol/l, in particular 20 to 500 mmol/l, in particular 20 to 200 mmol/l, in particular 50 to 200 mmol/l, in the volume of the polymer network present in the swollen state.

In a preferred embodiment, the polymer network has an average mesh size of 5 to 30 nm, in particular 7 to 30 nm.

In a preferred embodiment, the polymer network has in the process of its synthesis a solid content from 0,4 to 10, preferably from 1 to 9, preferably from 5 to 8, preferably 6 to 7 weight % (weight of polymer components based on overall weight).

In a particularly preferred embodiment, the coating is present in a swollen state, in particular in aqueous solution, in particular water, or buffered aqueous solution, in particular PBS, swollen form.

In a preferred embodiment, the polymer network, in particular the wound dressing, does not contain potassium ions, preferably no metal ions.

In a preferred embodiment, the polymer network, in particular the wound dressing, contains sodium ions, preferably contains sodium ions as the only metal ions.

In a preferred embodiment, the coating made from the polymer network has a thickness of at least 100 nm, preferably at least 110 nm.

In a preferred embodiment, the coating made from the polymer network has a thickness from 100 to 1200, preferably 110 to 1200, preferably 110 to 1000, preferably 200 to 900, preferably 300 to 800, preferably 300 to 700 nm.

The present invention in a preferred embodiment relates to a wound dressing, wherein the polymeric support is a nonwoven fabric, a woven fabric, a knitted fabric, a mesh, a fiber, a fiber mesh, a foam, in particular a polymeric foam, or a perforated film.

In a preferred embodiment the present wound dressing is in the form of a patch, a wound pflaster or bandage.

The present invention in a preferred embodiment relates to a wound dressing, wherein the mesh has a mesh size 500 µm to 2 mm, preferably 600 µm to 1,5 mm, preferably 700 µm to 1 mm.

The present invention in a preferred embodiment relates to a wound dressing, wherein the polymeric support is made of a synthetic polymer, in particular polyester, polyamide, cellulose, polyurethane, silicone polymer or polyethylene.

In a preferred embodiment, the polymeric support is a polyurethane foam.

In a preferred embodiment, the polymeric support is a polyester fiber mesh.

The present invention in a preferred embodiment relates to a wound dressing, wherein the wound dressing comprises a swellable polymer, in particular CMC (carboxymethyl cellulose), pectin or gelatin.

The present wound dressing in a preferred embodiment is biocompatible, but not bioabsorbable or degradable.

In a preferred embodiment, the polymer network is a synthetic polymer network.

In a preferred embodiment, the polymer network is hydratable.

In a preferred embodiment the present wound dressing does not contain any pharmaceutical substance.

In a preferred embodiment the present wound dressing does not contain any nitrates or nitrites or sources thereof.

In a preferred embodiment the present wound dressing does not contain any other components or substances, in particular no polymers, except the polymeric support and its coating made from the polymer network.

In a preferred embodiment the present wound dressing comprises at least one anti-microbial substance.

In a preferred embodiment the present wound dressing is non-adhesive.

In a preferred embodiment the present wound dressing comprises at least adhesive edge, preferably along at least one peripheral edge of the wound dressing.

In a preferred embodiment the present wound dressing is attached or attachable to a secondary wound dressing, preferably wherein the secondary wound dressing is attachable or attached distal to the wound that means the present wound dressing being located in between the wound and the secondary wound dressing.

The present invention also relates to a method for producing a wound dressing, in particular according to the present invention, comprising the following method steps:
a) providing at least one polymeric support, poly-(4-styrenesulfonic acid-co-maleic acid) and at least one uncharged polymer, in particular a functionalized uncharged polymer, or/and at least one non-polymeric crosslinker molecule, and optionally further components, in particular a reaction medium,
b) mixing the uncharged polymer or non-polymeric crosslinker molecule with the poly-(4-styrenesulfonic acid-co-maleic acid), preferably in a reaction medium; and
c) applying the mixture obtained in step b) to the polymeric support, thereby forming a polymer network of at least one poly-(4-styrenesulfonic acid-co-maleic acid) component and the at least one uncharged polymer component or/and a non-polymeric crosslinking component, so as to coat the polymeric support and obtain the wound dressing.

In a preferred embodiment of the present invention, the reaction medium is water, preferably deionised water.

In a preferred embodiment of the present invention, the applying step c) is a spray-coating step.

In a preferred embodiment of the present invention, the applying step c) is a casting step.

In a preferred embodiment, the, preferably functionalized, uncharged polymer has at least two functional groups, in particular end group, selected from the group consisting of amino group, thiol group, maleimide group, vinylsulfone group, acrylate group, carboxyl group and combinations thereof, wherein the at least two groups may be the same or different.

In a preferred embodiment, the, preferably functionalized, non-polymeric crosslinking molecule has at least two functional groups, in particular end group, selected from the group consisting of amino group, thiol group, maleimide group, vinylsulfone group, acrylate group, carboxyl group, hydroxylated aromatic group and combinations thereof, wherein the at least two groups may be the same or different.

Preferably provided in the at least one PSS-MA used in accordance with the invention and the at least one uncharged polymer and/or non-polymeric crosslinking molecule are at least two functional groups each selected from the group consisting of amino group, thiol group, maleimide group, vinylsulfone group, acrylate group, carboxyl group, and combinations thereof are each selected for the PSS-MA and the uncharged polymer and/or non-polymeric crosslinking molecule such that the functional groups of the reactants can form a covalent bond with each other between the PSS-MA and the at least one uncharged polymer or between the at least one PSS-MA and the at least one non-polymeric crosslinking molecule to form a network.

The uncharged polymers are preferably selected from the group consisting of poly(ethylene glycols) (PEG), poly(2-oxazolines) (POX), polyvinylpyrrolidones (PVP), poly(vinyl alcohols) (PVA) and/or polyacrylamides (PAM), which in a preferred embodiment have at least two or more functional groups, in particular amino groups, crosslinkable with the PSS-MA having carboxyl groups as a functional group.

The non-polymeric crosslinking molecules are preferably bifunctional crosslinking molecules which, in a preferred embodiment, have at least two or more functional groups, in particular amino groups, crosslinkable with the PSS-MA having carboxyl groups as a functional group.

In a particularly preferred embodiment, in step iii) the carboxyl groups of PSS-MA or non-polymeric crosslinking molecules containing them are activated 1-ethyl-3-(3-dimethylaminopropyl) carbodiimide/N-hydroxysulfosuccinimide (EDC/sNHS).

In a preferred embodiment the present wound dressing is for use in a therapeutic method for treating wounds, in particular wounds of the skin or mucosa, in particular acute or chronic wounds, in particular diabetic wounds, in particular lower leg ulcers, foot ulcers and pressure ulcers.

In a preferred embodiment the present wound dressing is for use in a therapeutic method for treating wounds in the exudation, granulation or epithelialization phase.

In a preferred embodiment the present invention relates to a use for treating wounds in a subject in need thereof, in particular a human or animal subject.

In a preferred embodiment the present invention relates to a method for treating wounds in a subject in need thereof, in particular a human or animal subject, wherein a wound dressing of the present invention is applied, in particular fixed to a wound, in particular a wound of the skin or mucosa. In a preferred embodiment, the wound dressing is removed from the wound once the loading capacity of the PSS-MA in respect to the chemokines removed from the wound has been reached.

In a preferred embodiment the present invention relates to a method for treating wounds in a subject in need thereof, in particular a human or animal subject, wherein a wound dressing of the present invention is applied, in particular fixed to a wound, in particular a wound of the skin or mucosa and a secondary wound dressing covering the wound dressing of the present invention is applied, preferably fixed, on the body-distal face of the present wound dressing.

The present wound dressing is preferably used after the wound has been cleaned and, if necessary, disinfected in a wound bed preparation procedure.

In the context of the present invention, the term "network" refers to a polymer network, comprising at least two polymer components as building units of said network, preferably at least two different polymer components, and wherein the polymer components are interconnected via chemical bonds with each other either directly or indirectly.

In the context of the present invention, the term "repeating unit" refers to a monomeric unit of a polymer. The term "at least one repeating unit" refers to the number of monomeric units making up the polymer and may be 1 to 100 000, preferably 10 to 10 000 or preferably 100 to 1 000 repeating units.

In the context of the present invention, the term "PSS-MA component or uncharged polymer component or non-polymeric crosslinking component" refers to the PSS-MA unit, uncharged polymer unit and crosslinking unit being integrally present in the molecular network of the polymer network. The polymer network components are building units of the polymer network and provided to be used in the process of the polymer network preparation in form of PSS-MA, non-polymeric crosslinkers and uncharged polymers. The term "at least one component" refers to the number of building units making up the polymer network and may be 1 to 100 000, preferably 10 to 10 000 or preferably 100 to 1000 building units.

The formula of the PSS-MA component showing the sulfonate (sulfonic acid) group and carboxylate (carboxylic acid) group is (with x and y each being an integer ≥1 of the repeating unit)

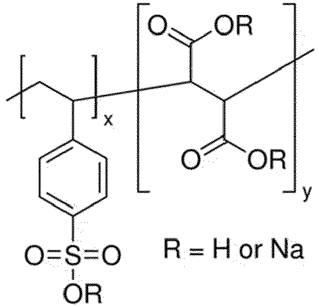

In the context of the present invention, the term "sulfonate group" refers to the anionic, deprotonated or to the protonated form of a sulfonic acid group, in particular to the anionic form.

In the context of the present invention, the term "chemokine" relates to cytokines or signaling proteins playing a major role in biological processes, including morphogenesis and wound healing. In particular, pro-inflammatory chemokines are considered herein, such as IL-1, IL-8, MCP, TNF-alpha, LPS, CXCL-8, CCL2, CCL3, CCL4, CCL5, CCL11 and CXCL10.

In the context of the present invention, the term "pharmaceutical substance" refers to a material, in particular compound, which exerts in a human or animal body a therapeutic, including prophylactic, action.

In the context of the present invention, the term "therapy" includes both a prophylactic and a curative treatment of an unhealthy state of a subject, in particular refers to a curative treatment.

In the context of the present invention, the term "use" being referred to in the context of a therapeutic method using the wound dressing means the offer, in particular, advertising, marketing, teaching, or prescription or sale or administration, in particular implantation, of the present wound dressing or a method using any one of it in a patient, such as evidenced for instance by prescription by a doctor, packing, packing leaflets, medical expert's information, expert's or doctor's or pharmacist's counselling, expert information in text or oral form, including in the internet, all of them also including off-label or cross-label use.

Where quantitative indications, in particular percentages, of components of a product or composition are given in the context of the present invention, these add up to 100% of the composition and/or product together with the other explicitly stated or expertly apparent further components of the composition or product, unless explicitly stated otherwise or expertly apparent.

In the context of the present invention, the term "a" or "the" is understood to refer to a single element or to a multitude of elements.

In the context of the present invention, the term "at least one" is understood to mean a quantity expressing a number of 1 or 2 or 3 or 4 or 5 or 6 or 7 or 8 or 9 or 10 and so on. In a particularly preferred embodiment, the term "at least one" may represent exactly the number 1. In another preferred embodiment, the term "at least one" may also mean 2 or 3 or 4 or 5 or 6 or 7.

In the context of the present invention, the term "composition" refers to a substance (in case a composition consisting of X is referred to) or to a mixture of substances (in case a composition comprising or containing or having or encompassing X is referred to).

If in context of the present invention a "presence", a "containing" or a "content" of a component is expressed, mentioned or implied in an amount of 0 [unit], in particular mg/kg, µg/kg or wt.%, this means that the respective components are not present in a measurable amount, in particular are not present.

The number of decimal places indicated corresponds to the precision of the measurement method used in each case.

If, in the context of the present invention, the first and second decimal places or the second decimal place are/is not indicated for a number, the latter is/are to be set as zero.

Where in the context of the present invention a "comprising", a "containing", an "encompassing", a "having" or a "content" of a component is expressly mentioned or implied, this means that the respective component is present, in particular present in a measurable amount.

In the context of the present invention, the terms "comprising", "encompassing" and "containing" are understood to mean that in addition to the elements explicitly covered by these terms, other elements not explicitly mentioned may be added. In the context of the present invention, it is also understood by these terms that only the explicitly mentioned elements are covered and that no further elements are present. In this particular embodiment, the meaning of the terms "comprising" and "containing" is synonymous with the term "consisting of", which term, that means "consisting of", means that the element referred to is the only element present, thereby excluding the presence of further elements. Furthermore, the terms "comprising", "encompassing" and "containing" also cover compositions which, in addition to the explicitly mentioned elements, also contain further elements which are not mentioned but which are of a functional and qualitatively subordinate nature. In this embodiment, the terms "comprising" and "containing" are synonymous with the term "consisting essentially of".

In the context of the present invention, the term "and/or" is understood to mean that all members of a group connected by the term "and/or" are represented both cumulatively with respect to each other in any combination, and alternatively with respect to each other. Exemplarily, for the expression "A, B and/or C" relating to members A, B and C of a group, the following disclosure is to be understood thereunder: i) (A or B or C), or ii) (A and B), or iii) (A and C), or iv) (B and C), or v) (A and B and C), or vi) (A and B or C), or vii) (A or B and C), or viii) (A and C or B). Thus, the term "and/or" is understood to refer to a cumulative combination of all members of the group, to any combinations of at least two and more members of said group and to each single member of the group. Thus, the term "and/or" is understood to be equivalent to the term "A or B or a combination thereof.

In the context of the present invention, individual components or constituents of a composition or of one of its components, determined quantitatively in relative form, in particular in percentages, preferably add up to 100% by weight of the respective composition referred to or of the composition or, if referred to, of a component thereof, unless otherwise stated.

Further preferred embodiments of the present invention are apparent from the dependent claims.

The present invention will be explained in more detail in the following examples which are not to be understood as limiting.

### Example 1: Regio-selective N-desulfation of heparin (Reference)

To synthesize regioselective N-desulfated heparin, heparin (MW 14 000 g/mol, manufacturer no. : 375095, Merck-Millipore, Germany) was first dissolved in deionized ultrapure water and desalted using an Amberlite IR-120 H⁺ ion exchange column (Sigma-Aldrich, Germany). A heparin-pyridine salt was formed by adding pyridine (Sigma-Aldrich, Germany) to the heparin solution until pH 6 was reached, which was concentrated by solvent evaporation in a B-490 rotary evaporator (Büchi, Germany), then lyophilized at -80°C (GEA Lyovac GT2, Germany) and stored at -20°C until further processing.

For the desulfation procedure, 1 g of heparin-pyridine was dissolved in a mixture of DMSO and deionized ultrapure water (95:5) and incubated at 50°C for 1.5 hours. The resulting solution was diluted 1:1 with deionized ultrapure water and adjusted to pH=9 with 1 M sodium hydroxide solution (Sigma-Aldrich, Germany). After dialyzing the solution (Spectrum Labs, Germany, MWCO= 8 kDa) against deionized ultrapure water for three days, N-acetylation of the desulfated heparin followed. For this purpose, 10% v/v methanol (Sigma-Aldrich, Germany) and 50 mM sodium carbonate (Sigma-Aldrich, Germany) were added to the solution. The acetylation reaction was carried out for three hours under cooling to 4°C and constant stirring at 400 rpm by adding 400 mit acetic anhydride (Sigma-Aldrich, Germany) per 1 mg heparin every half hour and then adjusting the pH=7.5 by adding a 2 M sodium carbonate solution. The sulfation degree have been analyzed using ¹H-NMR and elemental analysis as described in paper¹. Almost quantitative removal of N-sulfate and 6O groups have been confirmed.

### Example 2: Coating of fiber meshes with polyionic polymer networks

The following 9 different types (type 1 to type 9) of wound dressings have been fabricated (*Table 1*). The dressings of type 1-3 are based on a polyamide fiber mesh of the commercial wound contact layer Tegaderm Contact, whereas the types 4-9 are based on a fiber-mesh of polyester (Texinov, France). UrgoStart Contact is a commercial wound contact dressing of the company URGO medical, France serving as reference.

**Table 1: Overview of the types of wound dressing**

| | |
|---|---|
| **Type 1** | Polyamide fiber mesh coated with N-DSH-4armPEG polymer network |
| **Type 2** | Polyamide fiber mesh coated with PSS3M-4armPEG polymer network |
| **Type 3** | Polyamide fiber mesh coated with PSS1M-4armPEG polymer network |
| **Type 4** | Polyester fiber mesh coated with N-DSH-4armPEG polymer network |
| **Type 5** | Polyester fiber mesh coated with PSS3M-4armPEG polymer network |
| **Type 6-9** | Polyester fiber mesh coated with PSS1M-4armPEG polymer network |
| **UrgoStart Contact** | Polyester fiber mesh coated with Technology Lipido-Colloid- Nano Oligosaccharide Factor (TLC-NOSF) |

Types 1, 4 and UrgoStart Contact are reference examples.

The preparation of the different wound dressings (*Table 1*) was carried out by coating the fiber mesh support material with the polymer network containing one of the three different polyionic polymers (PIP) crosslinked with four-arm amine-terminated PEG (4-arm PEG, (MW 10 000 g/mol, Jenkem technology, USA). The polyionic polymers were either: 1) N-desulfated heparin derivative synthesized in-house by previously described procedures, see regioselective desulfation (example 1), 2) poly(4-styrenesulfonic acid-co- maleic acid) with the molar ratio 4-styrenesulfonic acid:maleic acid 3:1 (MW 20 000 g/mol, manufacturer no.: 434566, Sigma-Aldrich, Germany) (PSS3M) and 3) poly(4-styrenesulfonic acid-co- maleic acid) with the molar ratio 4-styrenesulfonic acid:maleic acid 1:1, MW 20 000 g/mol, manufacturer no.: 434558, Sigma-Aldrich, Germany, see Table 1) (PSS1M). The polyionic polymer types and the four-arm amine-terminated PEG, were used to form covalently crosslinked polymer networks around the fiber mesh by the aid of carbodiimide chemistry.

To synthesize the wound dressings (Type 1-6), the polymer network coating material was first mixed into a reaction mixture (details in Table 2). For that purpose, the respective polyionic polymer was dissolved in deionised ultrapure water at 4°C for 30 seconds at 500 rpm using a vortex mixer (IKA, Germany) (weights and volumes according to Table 2). In addition, 4-armPEG, 1-ethyl-3-(3-dimethylaminopropyl)carbodiimide (EDC; Sigma-Aldrich, Germany) and N-hydroxysulfosuccinimide (sulfo-NHS; Sigma-Aldrich, Germany) were dissolved in the same manner. To ensure complete dissolution of the polymer network building units, these solutions were additionally treated for 5 min at 4°C with an ultrasonic bath (RK 100H, Bandelin, Germany). To activate the carboxylic acid groups of the polyionic polymers (PIPs), EDC and sulfo-NHS) (s-NHS)(2:1 ratio of EDC:sulfo-NHS) were added to the dissolved PIP (4 moles sulfo-NHS, 8 moles EDC related per mole PIP of the final reaction mixture) and incubated at 4°C for the specified amount of time in Table 2. After the activation time of the PIPs, the dissolved amino-terminated 4-armPEG was also added and mixed for 30 seconds at 500 rpm with a vortex mixer (IKA, Germany). The final, fully mixed reaction mixture was afterwards used to coat the textile carrier (support) materials as indicated underneath.

**Table 2 Preparation parameters for the reaction mixture of the polymer network for the different wound dressing types (per 1 cm² textile support material), with respect to the PIP type, the weight of the polymers and carbodiimide activators, the volume of the ultrapure water as solvent and the activation time for the PIP with carbodiimide activators.**

| **Woun d dressi ng types** | **PIP** | **PIP MW [kD a]** | **PIP weigh t [mg]** | **4-arm PEG weigh t [mg]** | **EDC weigh t [mg]** | **NHS weigh t [mg]** | **EDC volu me [µl]** | **NHS volu me [µl]** | **PIP volu me [µl]** | **PEG volu me [µl]** | **Acti vati on time [min ]** |
|---|---|---|---|---|---|---|---|---|---|---|---|
| **Type 1** | N-DSH | 13.6 | 0.24 | 0.36 | 0.05 | 0.03 | 0.55 | 0.31 | 5.81 | 3.33 | 15 |
| **Type 2** | PSS3 M | 20 | 0.28 | 0.42 | 0.06 | 0.04 | 0.65 | 0.37 | 5.66 | 3.33 | 0.5 |
| **Type 3** | PSS1 M | 20 | 0.28 | 0.42 | 0.06 | 0.04 | 0.65 | 0.37 | 5.66 | 3.33 | 0.5 |
| **Type 4** | N-DSH | 13.6 | 0.1 | 0.14 | 0.02 | 0.01 | 0.22 | 0.12 | 2.32 | 1.33 | 15 |
| **Type 5** | PSS3 M | 20 | 0.12 | 0.12 | 0.02 | 0.01 | 0.18 | 0.1 | 2.38 | 1.33 | 0.5 |
| **Type 6** | PSS1 M | 20 | 0.12 | 0.12 | 0.02 | 0.01 | 0.18 | 0.1 | 2.38 | 1.33 | 0.5 |
| **Type 7** | PSS1 M | 20 | 0.12 | 0.12 | 0.02 | 0.01 | 0.18 | 0.1 | 2.38 | 1.33 | 0.5 |
| **Type 8** | PSS1 M | 20 | 0.06 | 0.06 | 0.01 | 0.005 | 0.09 | 0.05 | 1.19 | 0.665 | 0.5 |
| **Type 9** | PSS1 M | 20 | 0.03 | 0.03 | 0.005 | 0.003 | 0.045 | 0.025 | 0.595 | 0.333 | 0.25 |

The support materials were either: 1) the polyamide woven fabric 3M^{™} Tegaderm^{™} Non-Adherent Contact Layer (manufacturer's ref: 5642 NDC 8333-5642-01, 3M, Germany) or 2) a polyester knitted fabric, Texinov (reference number 555, Texinov medical textiles, France). The combination of the different polymer network coatings and the different support materials results in the different wound dressing types (Type 1-9) listed in *Table 1.* To produce the coated textile-based wound dressings of type 1-6, the respective activated polymer reaction mixture was used to coat the support materials by casting technique. Therefore, the volume of the polymer reaction mixture, indicated in Table 3 was transferred to the precut 1 cm² pieces of the support material and spread between two glass plates, in a sandwich of ethylene-chlorotrifluoroethylene copolymer films as non-adhesive molds (manufacturer No.: 916-989-91, Goodfellow, UK). This polymer coating amount results in a coating amount of 100 mL (for type 1, type 2 and type3) or 40 mL (for type 4, type 5 and type 6) per square metre of fiber mesh support material. To produce the coated textile-based wound dressing type 7, type 8 and type 9, a spray coating machine was used. The polymer precursors and crosslinkers (amounts per cm² shown in table 2) were dissolved as previously described. The PIP and sulfo-NHS were filled into a 30 ml syringes while 4armPEG and EDC were filled in another 30 mL syringe (BD Plastipak with Luer 30 ml, manufacturer no.: 301229 BD, Germany and BD Blunt fill needle 18G, manufacturer no.: 305181, BD, Germany). The polymer precursors and crosslinkers were afterwards mixed and pumped using syringe pump (PHD Ultra, Harvard Apparatus, USA) and sprayed using a commercial spray head (atomizing nozzle model SF8010SS, ExAir cooperation, USA) onto the polyester textile support. According to the volume of polymer mixture sprayed and the area of the fiber mesh, the coating amount was controlled between 40 mL (type 7), 20 mL (type 8) and 10 mL (type 9) per square meter fiber mesh support material.

Polymerization of the polymer network coating for all dressing types took place over a period of 12 hours where the EDC/NHS activated carboxylic acid groups of the PIPs react with the amine groups of the 4-arm PEG to form amide groups as stable, covalent crosslinks. Upon polymerization, the polymer network is formed surrounding the single textile fibers allowing for a stable coating. Subsequently, the coated wound dressings were swollen in phosphate-buffered saline for at least 3 hours prior to further use or characterization of the coated dressing materials. The commercially available wound dressing UrgoStart^{®} Contact (manufacturer no.: 100379, URGO medical, France) was also cut into 1 cm2 and used for comparison in the characterization of the wound dressings.

**Table 3: Preparation parameters for the coated wound dressing types, with respect to the type of the support material to be coated, the size of the support material per sample and the volume of activated polymer mixture used for the coating procedure.**

| **Wound dressing type** | **Support material type** | **Coated material surface area [cm²]** | **Activated polymer mixture volume [µl]** | **Polymer network coating amount [mL/m²]** | **Coating method** |
|---|---|---|---|---|---|
| **Type 1** | Polyamide textile | 1 | 10 | 100 | casting |
| **Type 2** | Polyamide textile | 1 | 10 | 100 | casting |
| **Type 3** | Polyamide textile | 1 | 10 | 100 | casting |
| **Type 4** | Polyester textile | 1 | 4 | 40 | casting |
| **Type 5** | Polyester textile | 1 | 4 | 40 | casting |
| **Type 6** | Polyester textile | 1 | 4 | 40 | casting |
| **Type 7** | Polyester textile | 1 | 4 | 40 | spray coating |
| **Type 8** | Polyester textile | 1 | 2 | 20 | spray coating |
| **Type 9** | Polyester textile | 1 | 1 | 10 | spray coating |
| **UrgoStart Contact** | / | 1 | / | / | / |

### Example 3: Preparation of chemokine containing simulated wound fluid

The chemokine scavenging performance of the wound dressings was analyzed by exposing the wound dressing to chemokine containing simulated wound fluid (SWF), a solution that closely mimics the salinity, and albumin concentration of human wound exudates^{2,3}. For that purpose, in all the binding experiments, the chemokines were added to SWF prepared by dissolving 5% bovine serum albumin (manufacturer no.: A7030, Sigma-Aldrich, Deutschland), 142 mM sodium chloride (Sigma-Aldrich, Deutschland), 2.5 mM calcium chloride (Sigma-Aldrich, Deutschland) and 0.05% Proclin (Sigma-Aldrich, Deutschland) in deionized, ultrapure water, on a plate shaker (VWR, Deutschland) at 300 rpm for one hour then sterile filtered using 0.2 µm syringe filter.

### Example 4: Determination of the chemokine-binding properties of wound dressing materials: Type 1, Type 2, Type 3 und UrgoStart^{®} Contact.

A chemokine containing incubation solution was prepared using chemokines typically present in wound exudate at physiologically relevant concentrations⁴. Therefore, 700 ng mL⁻¹ recombinant human Interleukin-8 (IL-8, manufacturer no.: 200-08M, Peprotech, Germany) and 1 ng mL⁻¹ recombinant human monocyte chemoattractant protein-1 (MCP-1, manufacturer no.: 300-04, Peprotech, Germany) were dissolved in SWF. The 1cm² wound dressings Type 1, Type 2, Type 3 und UrgoStart^{®} Contact were incubated with 0.25 mL of the chemokines containing SWF in a 24-well plate for 48 hours at room temperature on a plate shaker (VWR, Deutschland) at 100 rpm. Afterwards, the first chemokine solution was completely removed and fresh chemokines containing SWF was incubated with the same wound dressings. The cycle of chemokine repeated exposure was performed for three cycles. The total amount of chemokine introduced to the wound dressings over the three exposure cycles were equal to 525 ng of IL-8 and 750 pg of MCP-1. The amount of unbound protein in the collected supernatant was quantified by ELISA technique according to manufacturer's protocol (Human IL-8/CXCL8 DuoSet ELISA manufacturer no.: DY208 und Human CCL2/MCP-1 DuoSet ELISA manufacturer no.: DY279, R&DSystems, USA). The amount of unbound protein was subtracted from the original amount of protein used in the incubation solution, also determined by ELISA, to identify the amount of bound protein.

The cumulative amount of bound IL-8 and MCP-1 to the different dressings are displayed in Table 4. Surprisingly, Type 2 and Type 3 wound dressings bound ∼480 ng IL-8 and 745-750 pg MCP-1, and thus significant more than UrgoStart contact and the Type 1 wound dressing (see table 4).

**Table 4: Cumulative amount of IL-8 and MCP-1 bound following repeated exposure of the wound dressing to the chemokines containing simulated wound fluid (Mean ± standard deviation, n = 3)**

| | **UrgoStart Contact** | | **Type 1** | | **Type 2** | | **Type 3** | |
|---|---|---|---|---|---|---|---|---|
| | Mean [ng] | SD | Mean [ng] | SD | Mean [ng] | SD | Mean [ng] | SD |
| Cum. IL-8 bound [ng] | 221.92 | 71.48 | 387.57 | 35.62 | 481.19 | 3.31 | 481.69 | 4.42 |
| Cum. MCP-1 bound [pg] | 370.60 | 57.10 | 451.90 | 105.80 | 750.00 | 0.00 | 744.58 | 9.39 |

To analyze, if any saturation effects for chemokine binding might occur, i.e. if the chemokine binding capacity could be reached, the different wound dressings have been contacted with increasing amounts of the highly inflammatory chemokine IL-8. Therefore, wound dressings of Type 1, Type 2, Type3 und UrgoStart^{®} Contact of a sample size of 1 cm² each were incubated with 1 mL of SWF containing 100 ng, 500 ng or 1000 ng IL-8 in a 24-well plate for 24 hours at room temperature on a plate shaker (VWR, Deutschland) at 100 rpm. The amount of unbound protein was quantified by ELISA technique according to manufacturer's protocol. The amount of unbound protein was subtracted from the original amount of protein used in the incubation solution, also determined by ELISA, to identify the amount of bound protein.

The amounts of bound IL-8 for the different dressings (calculated as described above from the remaining concentration in the supernatant) are displayed in Table 5. While for UrgoStart Contact a saturation of 320-350 ng is reached upon exposure to 500 and 1000 ng IL-8 and with Type 1 wound dressing only a moderate increase in binding from 250 to 490 ng did occur upon 500 and 1000 ng IL-8 exposure, both types 2 and 3 (based on PIPs PSS3M and PSS1M) surprisingly showed no signs of saturation with 427 and 905 ng IL8 bound for type 2 and 418 and 718 ng IL-8 bound for type 3 for exposure of the dressings after contacting the dressings with an amount of 500 or 1000 ng IL-8 respectively.

**Table 5: Amount of IL-8 bound after incubation of the wound dressing in different amounts of IL-8 in simulated wound dressing for 24 hours. (Mean ± standard deviation, n = 3)**

| | **UrgoStart Contact** | | **Type 1** | | **Type 2** | | **Type 3** | |
|---|---|---|---|---|---|---|---|---|
| **IL-8 (ng)** | Mean [ng] | SD | Mean [ng] | SD | Mean [ng] | SD | Mean [ng] | SD |
| **100** | 57.26 | 16.21 | 73.55 | 15.57 | 86.25 | 5.38 | 84.67 | 3.32 |
| **500** | 354.32 | 24.13 | 250.95 | 32.05 | 416.82 | 59.47 | 417.63 | 42.46 |
| **1000** | 324.05 | 99.77 | 491.47 | 35.13 | 904.97 | 53.07 | 718.17 | 254.57 |

### Example 5: Determination of the chemokine-binding properties of wound dressing materials: Type 4, Type 5 and Type 6.

To highly challenge the chemokine-binding capacity of the polymer coating wound dressing, Type 4, Type 5 und Type 6 (40 mL/m² coating amount) were synthesized containing less than half the coating material of their respective Type 1, Type 2 und Type 3 dressings (100 mL/m² coating amount). The 1 cm² wound dressings were incubated in 1 mL incubation solution containing the IL-8 and MCP-1 in SWF at a concentration of 1000 ng/mL and 250 ng/mL in a 24-well plate for 48 hours at room temperature on a plate shaker (VWR, Deutschland) at 100 rpm. Afterwards, the chemokines containing SWF was completely removed and fresh chemokines containing SWF was incubated with the same wound dressings. The cycle of chemokine repeated exposure was performed for three cycles. The fourth cycle of chemokine exposure contained only IL-8 at a concentration of 1000 ng/mL. The sum amount of chemokine introduced to each wound dressing over the exposure cycles were equal to 4000 ng of IL-8 and 750 ng of MCP-1. The amount of unbound protein was quantified by ELISA technique according to manufacturer's protocol. The amount of unbound protein was then subtracted from the amount of unbound IL-8 after incubation with the uncoated polyester textile sample (negative control), also determined by ELISA, to identify the amount of bound protein for each dressing type.

The cumulative amount of bound IL-8 and MCP-1 to the different dressings are displayed in Table 6. Here again, the type 5 and type 6 (based on PIPs PSS3M and PSS1M) did clearly outperform the type 4 (based on the PIP NDSH) with respect to pro-inflammatory chemokine binding, as for IL-8 significant higher amount of 3944 ng (type 5) and 3938 ng (type 6) (both binding >98% IL-8) vs 3540 ng (for type 4, ∼88% IL-8 ) was bound and for MCP-1 a tremendously higher amount of 726/724 (type 5/6, binding >96% IL-8) vs. 572 ng (type 4, binding -75% MCP-1) was bound.

**Table 6: Cumulative amount of IL-8 and MCP-1 bound following repeated exposure of the wound dressing to the chemokines solutions in simulated wound fluid (Mean ± standard deviation, n = 3)**

| | **Type 4** | | **Type 5** | | **Type 6** | |
|---|---|---|---|---|---|---|
| | Mean [ng] | SD | Mean [ng] | SD | Mean [ng] | SD |
| **Cum. IL-8 bound [ng]** | 3540.1 | 30.1 | 3944.5 | 4.4 | 3938.3 | 5.7 |
| **Cum. MCP-1 bound [ng]** | 572.5 | 26.1 | 726.3 | 0.8 | 724.8 | 1.1 |

### Example 6: Estimation of the exudate transport through the wound dressing Type 6 and UrgoStart^{®} Contact.

The exudate transport through the wound dressing have been analyzed. Therefore, a series of PEG8000 solutions (manufacturer no.: 0159, VWR, Germany) with different viscosities (Table 7) were allowed to pass vertically through the dressing under the influence of gravity. For that purpose, the PEG8000 powder was completely dissolved in deionized, ultrapure water with concentrations according to Table 7. 1mL of the respective PEG8000 solutions was pipetted on a horizontally stretched wound dressing and the time required for the droplet of PEG8000 solution to vertically pass through the mesh, under the effect of gravity was recorded in Table 7. Type 6 wound dressing did clearly outperform the UrgoStart Contact with super-fast exudate transport of being a maximum of 1.25 s independent of the viscosity of the wound exudate-mimicking solution, while for UrgoStart Contact the exudate transport is significantly increasing upon 35-40 s upon viscosity increase to 325 mPa s.

**Table 7: Simulation of exudate transport; the time required for the PEG800 solution, with the respective viscosity, to vertically pass through the wound dressing under the effect of gravity (Mean ± standard deviation, n = 4)**

| | | **UrgoStart Contact** | | **Type 6** | |
|---|---|---|---|---|---|
| PEG8000 solution %w/v | PEG8000 solution viscosity [mPa.s] | Mean [s] | SD | Mean [s] | SD |
| 50% | 325 | 35 | 17.8 | 1.25 | 0.5 |
| 30% | 50.9 | 37.75 | 13.8 | 1.25 | 0.5 |
| 10% | 8.9 | 16.5 | 7.0 | 1.25 | 0.5 |
| 5% | 3.34 | 15.2 | 6.6 | 1.2 | 0.45 |
| 1% | 1.14 | 6.8 | 5.8 | 1.2 | 0.45 |
| 0% | 0.89 | 1 | 0 | 1 | 0 |

### Example 7: Determination of the chemokine-binding properties of spray-coated wound dressing materials: Type 7, Type 8 and Type 9

The spray coating technique, used to prepare type 7-9 dressings, is most beneficial for upscaling of the production process and to precisely control the amount of polymer network coating at the textile support. Furthermore, a more homogenous coating have been surprisingly achieved with the spray coating technique compared to the casting technique of type 1-6 dressing. Type 7 dressing possessed a polymer network coating amount of 40 mL/m², similar to Type 6 dressing. Type 8 and type 9 dressings were produced with reduced polymer network coating amounts of 20 mL/m² and 10 mL/m² respectively. To evaluate the reproducibility of the spray-coating method, a high number of samples (>25) were tested and showed a good reproducibility of the polymer network coating amount.

Subsequently, the 1 cm² wound dressing samples were incubated in 1 mL incubation solution containing the IL-8 in SWF at a concentration of 200 ng/mL in a 24-well plate for 24 hours at room temperature on a plate shaker (VWR, Deutschland) at 100 rpm. Afterwards, the chemokines containing SWF was collected and quantified by ELISA technique according to manufacturer's protocol, to determine the amount of unbound protein. The amount of unbound protein was then subtracted from the amount of unbound IL-8 after incubation with the uncoated polyester textile sample (negative control), also determined by ELISA, to identify the amount of bound protein for each dressing type. Surprisingly, the dressings type 7, type 8 and type 9, formed by spray coating method, were able to effectively bind the IL-8 from the SWF, proving that the spray coating method is capable of producing a stable and reproducible coating on the textile carrier. Interestingly, the polymer coating amount of 40 mL/m² (type 7) resulted in 187 ng IL-8 bound and reducing the coating amount to 50% (20 mL/m² (type 8)) a similar amount of 189 ng have been bound (>90% IL-8 bound for both types of dressings). Further reduction of the polymer network coating to 10 mL/m² still resulted in considerable binding of the IL-8 of >75% (152.1 ng), indicating an unexpected high IL-8 scavenging capacity of the polymer coating at minimal polymer network coating amount that is economically beneficial.

**Table 8 Amount of IL-8 bound after incubation of the wound dressing in simulated wound fluid containing IL-8 for 24 hours. (Mean ± standard deviation, n >25)**

| | **Type 7** | | **Type 8** | | **Type 9** | |
|---|---|---|---|---|---|---|
| | Mean [ng] | SD | Mean [ng] | SD | Mean [ng] | SD |
| **IL-8 bound [ng]** | 186.6 | 2.1 | 189.2 | 1.8 | 152.1 | 23.5 |

### References:

1. Atallah, P. et al. In situ- forming, cell-instructive hydrogels based on glycosaminoglycans with varied sulfation patterns. Biomaterials 181, 227-239 (2018).
2. Trengove, N. J. et al. Analysis of the acute and chronic wound environments: the role of proteases and their inhibitors. Wound Repair Regen. 7, 442-52 (1999).
3. Eming, S. A. et al. Differential proteomic analysis distinguishes tissue repair biomarker signatures in wound exudates obtained from normal healing and chronic wounds. J. Proteome Res. 9, 4758-4766 (2010).
4. Lohmann, N. et al. Glycosaminoglycan-based hydrogels capture inflammatory chemokines and rescue defective wound healing in mice. Sci. Transl. Med. 9, (2017).

## Claims

1. A wound dressing comprising a polymeric support having a coating of a polymer network, the polymer network comprising at least one poly-(4-styrenesulfonic acid-co-maleic acid) component covalently bonded to at least one of at least one uncharged polymer component and at least one non-polymeric cross-linking component thereby forming the polymer network, wherein the at least one poly-(4-styrenesulfonic acid-co-maleic acid) component comprises sulfonate groups.

2. The wound dressing of claim 1, wherein the polymer network is a synthetic polymer network.

3. The wound dressing of claim 1 or 2, wherein the polymer network is hydratable.

4. The wound dressing of any one of the preceding claims, wherein the at least one uncharged polymer component is selected from the group consisting of poly(ethylene glycol) (PEG) component, poly (2-oxazoline) (POX) component, polyvinylpyrrolidone (PVP) component, poly(vinyl alcohol) (PVA) component, polyacrylamide (PAM) component, and combinations thereof.

5. The wound dressing according to any one of the preceding claims, wherein the at least one uncharged polymer component is a poly(ethylene glycol) component, in particular a linear or multi-arm, in particular four- or eight-arm, poly(ethylene glycol) component.

6. The wound dressing of any one of the preceding claims, wherein the at least one non-polymeric cross-linking component is selected from the group consisting of ethylenediamine, propylenediamine (1,3-diaminopropane), butane-1,4-diamine, pentane-1,5-diamine (cadaverine), hexamethylene-1,6-diamine oxalic acid, malonic acid, succinic acid, glutaric acid, adipic acid, pimelic acid, N-(2-aminoethyl) maleimide, enzymatically cleavable peptides, and combinations thereof.

7. The wound dressing according to any one of the preceding claims, wherein the polymeric support is a nonwoven fabric, a woven fabric, a knitted fabric, a mesh, a fiber, a fiber mesh, a foam, in particular a polymeric foam, or a perforated film.

8. The wound dressing according to any one of the preceding claims, wherein the polymeric support is made of a synthetic polymer, in particular polyester, polyamide, cellulose, polyurethane, silicone polymer or polyethylene.

9. The wound dressing according to any one of the preceding claims, wherein the wound dressing comprises a swellable polymer, in particular CMC, pectin or gelatin.

10. The wound dressing according to any one of the preceding claims, wherein the mesh has a mesh size 500 µm to 2 mm.

11. The wound dressing according to any of the preceding claims, wherein the wound dressing, in particular the coating, is non-adhesive.

12. The wound dressing according to any one of the preceding claims, wherein the wound dressing comprises at least one anti-microbial agent.

13. The wound dressing according to any one of the preceding claims for use in a therapeutic method for treating wounds, in particular wounds of the skin or mucosa, in particular acute or chronic wounds, in particular diabetic wounds, in particular lower leg ulcers, foot ulcers and pressure ulcers.

14. The wound dressing according to any one of the preceding claims for use in a therapeutic method for treating wounds in the exudation, granulation or epithelialization phase.

15. A method for producing a wound dressing, in particular according to any one of claims 1 to 14, comprising the following method steps:
a) providing at least one polymeric support, poly-(4-styrenesulfonic acid-co-maleic acid) and at least one uncharged polymer, in particular a functionalized uncharged polymer, or/and at least one non-polymeric crosslinker molecule, and optionally further components, in particular a reaction medium,
b) mixing the uncharged polymer or non-polymeric crosslinker molecule with the poly-(4-styrenesulfonic acid-co-maleic acid), preferably in a reaction medium; and
c) applying the mixture obtained in step b) to the polymeric support, thereby forming a polymer network of at least one poly-(4-styrenesulfonic acid-co-maleic acid) component and the at least one uncharged polymer component or/and a non-polymeric crosslinking component, so as to coat the polymeric support and obtain the wound dressing.
